# EUROPEAN PATENT SPECIFICATION

(11) **EP 0 785 186 B1**
(45) Date of publication and mention of the grant of the patent: **31.05.2000**
(21) Application number: 97300354.4
(22) Date of filing: 21.01.1997
(51) Int. Cl.: C07C 253/30, C07C 255/47, C07C 255/46

(54) **Process for preparing diels-alder addition product from conjugated diolefin and acrylonitrile**
Verfahren zur Herstellung von Diels-Alder-Additionsprodukten aus konjugierten Diolefinen und Acrylnitril
Procédé de préparation de produits d'addition d'une réaction de Diels-Alder à partir d'une oléfine conjugée et acrylonitrile

(30) Priority: 22.01.1996 JP 801796; 22.01.1996 JP 801896
(43) Date of publication of application: 23.07.1997
(73) Proprietor: MITSUI CHEMICALS, INC., Tokyo (JP)
(72) Inventor: Inomata, Masamitsu, Takaishi-shi, Osaka (JP); Takeno, Masahiro, Mobara-shi, Chiba (JP); Numa, Akio, Mobara-shi, Chiba (JP); Mizutani, Hiroki, Mobara-shi, Chiba (JP); Ebina, Masanobu, Yokohama-shi, Kanagawa (JP); Fukada, Isao, Mobara-shi, Chiba (JP)
(74) Representative: Nicholls, Kathryn Margaret

(56) References cited:
- DE-A- 1 518 552
- DE-A- 2 432 630

## Description

The present invention relates to a process for preparing a Diels-Alder addition product of a conjugated diolefin and acrylonitrile by means of reacting the conjugated diolefin and acrylonitrile.

It is known to prepare a Diels-Alder addition product of a conjugated diolefin and acrylonitrile by means of Diels-Alder reaction of the conjugated diolefin and acrylonitrile. This reaction proceeds thermally and is thus desirably conducted at an elevated temperature. It is, however, known that increased amounts of polymers are contained in the reaction solution when the reaction is carried out at a high temperature.

These polymers adhere to or are deposited on inner surfaces at or around the feed inlet and the outlet of the reaction solution as the polymers are insoluble in the reaction solution. This interferes with the operation of the reaction. Processes have therefore been proposed that add various polymerization inhibitors to inhibit by-production of the polymers and thereby to avoid the above mentioned problems. In order to avoid polymerization of a diolefin or olefin used for the Diels-Alder reaction, the following processes are known: Japanese Patent Publication No. 54-9198 discloses the use of an N-nitrosoamine compound, Japanese Patent Laid-Open No. 61-165338 discloses the use of an alkylphenol compound, Japanese Patent Publication No. 57-7131 discloses the use of a *p*-phenylenediamine compound, and Japanese Patent Laid-Open No. 62-167733 discloses the use of a hydroxylamine compound.

Alternatively, as a process for preparing cyanonorbornene through the Diels-Alder reaction between acrylonitrile and the conjugated diolefin which is produced from dicyclopentadiene, Japanese Patent Laid-Open No. 7-188147 discloses performing the reaction in the presence of an N-nitrosoamine compound and Japanese Patent Publication No. 59-51533 discloses addition of cyanonorbornene in advance to a raw material of the reaction.

Japanese Patent Laid-Open No. 51-34139 proposes a process in which the raw material is kept in a liquid phase by feeding the raw material into a reaction solution through a lower portion of the reactor and taking out the reaction solution through an upper portion of the reactor to achieve reaction under a pressure higher than the pressure generated spontaneously during the reaction at a given temperature, as a process for improving a yield of cyanonorbornene while reducing the amount of by-produced polymers in the reaction between dicyclopentadiene and acrylonitrile.

Studies made by the present inventors revealed that the reaction between a conjugated diolefin and acrylonitrile may tend to produce a highly insoluble polymer in a reactor or in a pipe or may sometimes be accompanied with danger, depending on the methods and conditions used.

More specifically, the present inventors carried out reactions under various conditions by using a so-called tubular reactor in which the conjugated diolefin and acrylonitrile are heated and reacted in a spiral pipe, or using a vessel type reactor. As a result, in the so-called tubular reactor in which the conjugated diolefin and acrylonitrile are reacted in the pipe, insoluble polymers adhere to the inner surface of the pipe and the pipe may then be clogged with such polymers. This process is hardly applicable to continuous production. On the other hand, the vessel type reactor does not suffer from adhesion of the insoluble polymers to the inner surface thereof when the reaction is carried out with the reactor all filled with a solution. The present inventors have found that this process involves considerable danger because of sudden changes of pressure in the reactor, and that the insoluble polymers adhere to pipes through which the raw material is supplied to the reaction solution or to a discharge portion of the pipes with the possibility of clogging the pipes after all. The process described in the above mentioned Japanese Patent Laid-Open No. 51-34139 is thus revealed to be effective as a process for improving the yield of cyanonorbornene while reducing the by-produced amount of the polymers but to be a far from safe reaction.

As described above, there are some conventional processes directed towards inhibiting production of any insoluble polymers in the reaction solution during the reaction. However, the structure of, or conditions in a reactor where production of the insoluble polymers can be inhibited effectively have not been described, nor has a process for carrying out reaction in a safe manner.

An object of the present invention is to provide a process for preparing a Diels-Alder addition product of a conjugated diolefin and acrylonitrile applicable to industrial fields in which the reaction can be carried out in a safe manner and production of any insoluble polymers can be inhibited effectively in preparing the Diels-Alder addition product from the conjugated diolefin and acrylonitrile by means of Diels-Alder reaction between the conjugated diolefin and acrylonitrile.

More specifically, the present invention provides a process for preparing a Diels-Alder addition product of a conjugated diolefin and acrylonitrile comprising the steps of: providing a vessel type reactor; increasing pressure in a gaseous phase present therein by using a gas inert to the reaction; releasing a premixed solution of the conjugated diolefin,or a compound which produces the conjugated diolefin in the reactor, and acrylonitrile through an upper portion of the reactor into the gaseous phase and dropping the mixed solution into a liquid phase to react; reacting the conjugated diolefin and acrylonitrile at a temperature of 180°C to 200°C and removing reaction product from a lower portion of the reactor. This process can facilitate long-time continuous operation in a safe manner.

Embodiments of the invention are described below by way of example only and with reference to the drawings of which:
Fig. 1 is a view for use in describing a reactor used in Examples 1 through 12, 14 and 15, and Comparative Examples 1, 2 and 7;
Fig. 2 is a schematic view for describing the embodiment in Comparative Examples 3;
Fig. 3 is a schematic view for describing the embodiments of Comparative Examples 4 and 9;
Fig. 4 is a schematic view for describing the embodiments of Comparative Examples 5 and 8;
Fig. 5 is a schematic view for describing the embodiment of Comparative Examples 6; and
Fig. 6 is a schematic view for describing the embodiment of Example 13.

In the figures, reference numerals and symbols represent the following:
1: a raw material solution, 2: a metering pump, 3: a reactor, 4: a reaction solution, 5: an electrical heater, 6: an automatic control valve, 7: a solution of a Diels-Alder addition product from conjugated diolefin and acrylonitrile, 8: an equalizing pipe, 9: a gas-liquid separator, 10: a heat medium, A: a stirrer, P: pressure gauge, T: a thermometer, and L: a differential pressure type level gauge.

In the present invention, the vessel type reactor to be used for the reaction between a conjugated diolefin and acrylonitrile is not limited specifically and any vessel may generally be used that is capable of holding liquid therein and of being sealed. However, a typical reactor is cylindrical, spherical or reverse conical, or a combination of these.

In the present invention, the reaction between the conjugated diolefin and acrylonitrile is carried out in a liquid phase. A gaseous phase is provided in the reactor described above and is pressurized by a gas which is inert to the reaction. In this event, an excessively small volume of the gaseous phase is accompanied with considerable danger because the pressure in the reactor tends to be changed or fluctuated suddenly. By contrast, an excessively large volume of the gaseous phase may be disadvantageous with respect to volume efficiency. In this respect, it is preferable that the volume of the gaseous phase is within the range of 10-50% relative to the entire volume of the reactor. It is more preferable that the reaction is carried out with the volume of the gaseous phase relative to the entire volume of the reactor being within the range of 20-40%.

The pressure during the reaction in the present invention is preferably within the range of 6-70 kg/cm²·G depending on the kind of the conjugated diolefin used or the kind of compound from which the conjugated diolefin is produced in the reactor. The gas which is inert to the reaction, and which is used for increasing the pressure may be any gas that has no reactivity towards the raw materials and reaction products and which does not absorb them to a considerable degree, though (the choice of gas depends) on the kind of the conjugated diolefin used or of the compound from which the conjugated diolefin is produced in the reactor. More specifically, the gas used may be at least one selected from the group consisting of nitrogen, argon, helium, methane, ethane, propane, n-butane, and isobutane. Of these, nitrogen can typically be used advantageously because it is readily available. A satisfactory effect can be obtained with nitrogen.

For example, the pressure during the reaction is preferably within the range of 6-30 kg/cm²·G, and more preferably within the range of 6-10 kg/cm²·G when the compound from which the conjugated diolefin is produced is dicyclopentadiene.

It is also preferable that the reaction is carried out at a pressure ranging from 50 to 70 kg/cm²·G and that the inert gas used for increasing the pressure is at least one selected from the group consisting of nitrogen, argon, helium, methane and ethane, when the conjugated diolefin is butadiene.

Furthermore, it is preferable that the reaction is carried out at a pressure ranging from 30 to 50 kg/cm²·G and that the inert gas used for increasing the pressure is at least one selected from the group consisting of nitrogen, argon, helium, methane, ethane and propane, when the conjugated diolefin is isoprene.

In the present invention, the conjugated diolefin or the compound from which the conjugated diolefin is produced in the reactor and acrylonitrile, which are raw materials, are mixed in advance and the resultant mixed solution is released through an upper portion of the reactor into the gaseous phase to continuously feed the raw material and is dropped into the liquid phase to achieve the reaction. This is a significantly important factor for preparing continuously addition products of the conjugated diolefin and acrylonitrile in a stable manner for a long time without producing any polymers in a pipe through which the raw material is supplied. On the other hand, the reaction product is taken out through a lower portion of the reactor.

In this event, if the conjugated diolefin or the compound from which the conjugated diolefin is produced in the reactor and acrylonitrile, which are the raw materials, are fed to the reactor separately rather than being mixed in advance, an insoluble polymer tends to adhere to surfaces at or around the inlet for the raw materials, especially for acrylonitrile, of the reactor. If the pipe through which the mixed solution of the conjugated diolefin, or the compound from which the conjugated diolefin is produced in the reactors, and acrylonitrile is fed is introduced into the reaction solution for the reaction, a large amount of insoluble polymers adheres to the pipe. In either case, the pipe is likely to be clogged with such insoluble polymers. Therefore, it is very difficult to prepare continuously addition products of a conjugated diolefin and acrylonitrile for a long time in a stable manner.

In the present invention, the reaction between the conjugated diolefin , or the compound from which the conjugated diolefin is produced in the reactor, and acrylonitrile is typically carried out in the presence of a compound that inhibits production of a polymer in order to inhibit by-production of polymers in the reaction solution. The compound to inhibit the production of the polymers may be any one of various compounds as used for typical Diels-Alder reaction. Of these, it is particularly preferable in the present invention to use N-nitrosoamine compound or *p*-phenylenediamine compound. These compounds are capable of inhibiting very effectively production of the soluble polymer which is a precursor of the insoluble polymer that otherwise is produced during the reaction between the conjugated diolefin, or the compound from which the conjugated diolefin is produced in the reactor, and acrylonitrile.

Preferable examples of the N-nitrosoamine compound include N-nitrosodimethylamine, N-nitrosodiethylamine, N-nitrosomethylethylamine, N-nitrosodiphenylamine, N-nitrosobenzylaniline and 4-nitroso-N,N-dimethylaniline. Preferable examples of the *p*-phenylenediamine compound include N-phenyl-N'-isopropyl-*p*-phenylenediamine. These compounds may be used alone or as a mixture of two or more thereof.

The amount of the compound that inhibits the production of polymers is typically within the range of 0.003-1% by weight, and preferably within the range of 0.005-0.3% by weight relative to the total amount of the conjugated diolefin, or the compound from which the conjugated diolefin is produced in the reactor,and acrylonitrile, both of which are reactants. The compound that inhibits the production of polymers is merely required to be present in the reaction solution during the reaction. For example, the compound may be added in advance to the conjugated diolefin or the compound from which the conjugated diolefin is produced in the reactor. Alternatively, the compound may be added to the mixture of the conjugated diolefin or the compound from which the conjugated diolefin is produced in the reactor and acrylonitrile.

In the present invention, the temperature for the reaction between the conjugated diolefin and acrylonitrile is 180-200°C, more preferably 185-200°C, and most preferably 190-200°C. A reaction temperature lower than 180°C reduces the yield of the resultant addition product of the conjugated diolefin and acrylonitrile and there remains a large amount of unreacted materials in the reaction solution, though the production of the soluble polymer can be inhibited. On the other hand, a reaction temperature of higher than 200°C causes significant production of soluble polymers in the reaction solution.

In the present invention, the reaction between the conjugated diolefin and acrylonitrile, which are the raw materials, is, in theory, the reaction between 1 mole of conjugated diolefin and 1 mole of acrylonitrile. However, it is preferable that the reaction be carried out in 1-1.5 moles of acrylonitrile relative to 1 mole of conjugated diolefin. If the amount of acrylonitrile is smaller than 1 mole this increases production of by-products while amounts larger than 1.5 moles cause a large amount of unreacted acrylonitrile to remain. These amounts are not preferable because their use may complicate purification. Accordingly, it is preferable that the reaction is carried out by using, for example, 2-3 moles of acrylonitrile relative to 1 mole of dicyclopentadiene when the latter is used as the compound from which the conjugated diolefin is produced in the reactor.

Though depending on the reaction temperature, the reaction time may typically be within the range of 0.1-6 hours in order to obtain a high yield of the addition product while bearing the productivity thereof in mind. The reaction time may be shorter or longer than the one described above, if necessary.

For the implementation of the process according to the present invention, the reaction is typically carried out continuously by feeding continuously a mixed solution of the conjugated diolefin, or the compound from which the conjugated diolefin is produced in the reactor, and acrylonitrile, which are the raw materials, into the reactor using, for example, a metering pump, and supplying the inert gas to the reaction to adjust the pressure. However, it is also effective in a batch process to carry out the reaction at a higher pressure applied by the inert gas to the reaction. For the continuous process, the conjugated diolefin or the compound from which the conjugated diolefin is produced in the reactor and acrylonitrile are mixed in advance and supplied to the reactor rather than being supplied thereto separately.

### EXAMPLES

Advantages of the present invention are described below in conjunction with a set of examples and comparative examples.

### Example 1

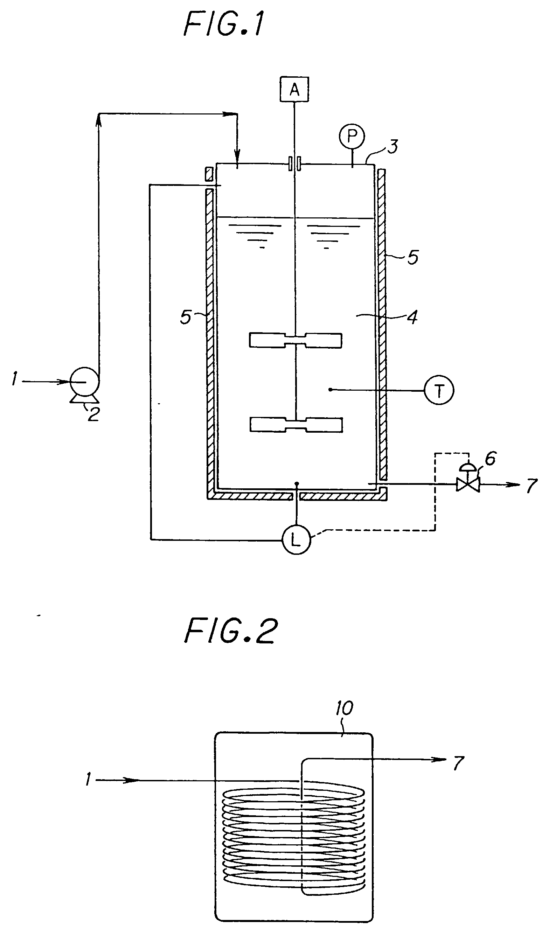

A reactor used was a pressure autoclave made of stainless steel and equipped with a stirrer having the inner volume of 1,500 ml, the essentials of which are shown in Fig. 1. Before initiation of reaction, 1,015 ml of cyanonorbornene was filled in the autoclave. The content of the reactor was heated to 170°C while stirring at the rotating speed of 600 rpm. Next, dicyclopentadiene and acrylonitrile were mixed in a molar ratio of 1:2.5. N-nitrosodiphenylamine was added to the mixed solution such that the content thereof is 0.1% by weight to provide a raw material solution. The raw material solution was continuously supplied to the autoclave by means of a metering pump such that the residence time becomes 4 hours. During the reaction, the liquid level in the reactor was kept such that the volume of the gaseous phase over the reaction solution in the reactor is 30% relative to the entire volume of the reactor. In addition, nitrogen gas was fed to the reactor to adjust the pressure so as to be 8 kg/cm²·G in the autoclave. Furthermore, the temperature of the liquid was kept to 190°C for the continuous reaction.

As a result, cyanonorbornene was continuously obtained with the average yield of 93% by mole based on cyclopentadiene obtained by decomposition of dicyclopentadiene used as the raw material. In addition, the amount of the soluble polymer produced in the reaction solution immediately after the initiation of the reaction was 0.002% by weight, which did not change significantly in 60 days after the initiation of the operation. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

Gas chromatography was used for the analysis of the reaction solution. The amount of the soluble polymer produced was determined by means of diluting the reaction solution with tetrahydrofuran and subjecting the resultant solution to gel permeation chromatography.

### Example 2

Example 1 was repeated except that the liquid temperature during the reaction was kept at 200°C. As a result, cyanonorbornene was continuously obtained with the average yield of 96% by mole. In this example, the amount of the soluble polymer produced was 0.003% by weight at the beginning of the reaction, which did not change significantly in 60 days after the initiation of the reaction. In addition, the operation was stopped after 80 days from the initiation to inspect in detail the inlet for the raw material, the inner wall, the outlet for the reaction solution and the stirrer of the autoclave. As a result, no insoluble polymer was found to be adhered.

### Example 3

Example 1 was repeated except that N-nitrosodiethylamine nitrosodiethylamine was used in place of and was added to the mixed solution of dicyclopentadiene and acrylonitrile to be 0.005% by weight. As a result, cyanonorbornene was continuously obtained with the average yield of 93% by mole. In this example, the amount of the soluble polymer produced was 0.01% by weight at the beginning of the reaction, which did not change significantly in 60 days after the initiation of the reaction. In addition, the operation was stopped after 80 days from the initiation to inspect in detail the inlet for the raw material, the inner wall, the outlet for the reaction solution and the stirrer of the autoclave. As a result, no insoluble polymer was found to be adhered.

### Example 4

Example 1 was repeated except that the liquid temperature during the reaction was kept at 180°C. As a result, cyanonorbornene was continuously obtained with the average yield of 87% by mole. In this example, the amount of the soluble polymer produced was 0.001% by weight at the beginning of the reaction, which did not change significantly in 20 days after the initiation of the reaction.

### Example 5

Example 1 was repeated except that N-phenyl-N'-isopropyl-*p*-phenylenediamine was used in place of N-nitrosodiphenylamine in the same amount. As a result, cyanonorbornene was continuously obtained with the average yield of 93% by mole. In this example, the amount of the soluble polymer produced was 0.15% by weight at the beginning of the reaction, which did not change significantly in 60 days after the initiation of the reaction. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

### Example 6

Example 1 was repeated except that 4-nitroso-N,N-dimethylaniline was used in place of N-nitrosodiphenylamine in the same amount and that the nitrogen gas was supplied to adjust the pressure in the autoclave to be 7 kg/cm²·G. As a result, cyanonorbornene was continuously obtained with the average yield of 93% by mole. In this example, the amount of the soluble polymer produced was 0.13% by weight at the beginning of the reaction, which did not change significantly in 60 days after the initiation of the reaction. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

### Example 7

Example 1 was repeated except that the raw material solution was supplied in a manner that the residence time was 1 hour. As a result, cyanonorbornene was continuously obtained with the average yield of 90% by mole. In this example, the amount of the soluble polymer produced was 0.001% by weight at the beginning of the reaction, which did not change significantly in 60 days after the initiation of the reaction. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

### Example 8

Example 1 was repeated except that the volume of the gaseous phase over the reaction solution in the reactor was kept to be 20% relative to the entire volume of the reactor. As a result, cyanonorbornene was continuously obtained with the average yield of 93% by mole. In this example, the amount of the soluble polymer produced was 0.002% by weight at the beginning of the reaction, which did not change significantly in 60 days after the initiation of the reaction. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

### Example 9

Example 1 was repeated except an argon gas was used in place of the nitrogen gas to adjust the pressure. As a result, cyanonorbornene was continuously obtained with the average yield of 93% by mole. In this example, the amount of the soluble polymer produced was 0.002% by weight at the beginning of the reaction, which did not change significantly in 60 days after the initiation of the reaction. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

### Example 10

Example 1 was repeated except that a methane gas was used in place of the nitrogen gas to adjust the pressure. As a result, cyanonorbornene was continuously obtained with the average yield of 92% by mole. In this example, the amount of the soluble polymer produced was 0.003% by weight at the beginning of the reaction, which did not change significantly in 60 days after the initiation of the reaction. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

### Example 11

Example 1 was repeated except that a propane gas was used in place of the nitrogen gas to adjust the pressure. As a result, cyanonorbornene was continuously obtained with the average yield of 91% by mole. In this example, the amount of the soluble polymer produced was 0.003% by weight at the beginning of the reaction, which did not change significantly in 60 days after the initiation of the reaction. In addition, the operation was stopped after 70 days from the initiation to inspect in detail the inlet for the raw material, the inner wall, the outlet for the reaction solution and the stirrer of the autoclave. As a result, no insoluble polymer was found to be adhered.

### Example 12

Example 1 was repeated except that an *n*-butane gas was used in place of the nitrogen gas to adjust the pressure. As a result, cyanonorbornene was continuously obtained with the average yield of 90% by mole. In this example, the amount of the soluble polymer produced was 0.005% by weight at the beginning of the reaction, which did not change significantly in 40 days after the initiation of the reaction. In addition, the operation was stopped after 50 days from the initiation to inspect in detail the inlet for the raw material, the inner wall, the outlet for the reaction solution and the stirrer of the autoclave. As a result, no insoluble polymer was found to be adhered.

### Comparative Example 1

Example 1 was repeated except that the reaction was conducted under the pressure of 4-5 kg/cm²·G, which was generated spontaneously in the reactor without supplying the nitrogen gas. As a result, the amount of the soluble polymer produced was 0.003% by weight at the beginning of the reaction. The operation was stopped after 30 days from the initiation to inspect in detail the inside the autoclave. As a result, insoluble polymers were adhered to the inlet for the raw material and the inner wall.

### Comparative Example 2

Example 1 was repeated except that the liquid temperature during the reaction was kept at 210°C and the reaction was conducted under the pressure of 9 kg/cm²·G adjusted with nitrogen gas. As a result, the amount of the soluble polymer produced was 0.01% by weight at the beginning of the reaction, which increased to 1% in 5 days after the initiation of the operation. The operation was stopped after 10 days from the initiation because it became difficult to supply the raw material. The inspection of the inside of the autoclave revealed that a significant amount of insoluble polymers were adhered to the inlet for the raw material and the inner wall.

### Comparative Example 3

A reactor used was a stainless pipe having the inner diameter of 3 mm and the length of 4 m, which was formed into a spiral of a number of coils, and of which essentials are shown in Fig. 2. The same raw material solution as prepared in Example 1 was passed through the pipe. The temperature of the oil outside thereof, which served as the heat medium, was increased to 190°C and the reaction was carried out continuously with the residence time of 4 hours.

In this comparative example, it became difficult to supply the raw material in 1 day after the initiation of the reaction, so that the operation was stopped at that time. The inside of the tube or the reactor was inspected to find that a large amount of insoluble polymers adhered thereto and the pipe was just being clogged.

### Comparative Example 4

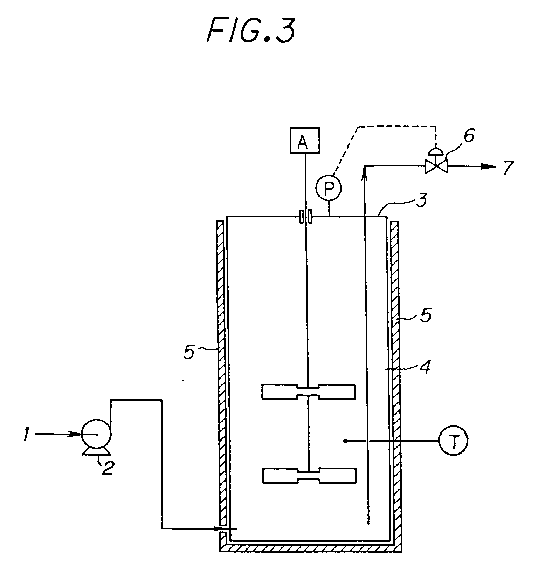

Example 1 was repeated except that the reactor of which essentials are shown in Fig. 3 was used and that the reactor was all full of the reaction solution to prepare cyanonorbornene. However, this example was suffered from considerable fluctuation of the pressure in the reactor with increase of the inner temperature. The pressure was suddenly fluctuated in the range of 5-30 kg/cm²·G. There was a great possibility of damaging the reactor and auxiliary facilities. It was thus found to be difficult to prepare cyanonorbornene continuously in a safe manner.

### Comparative Example 5

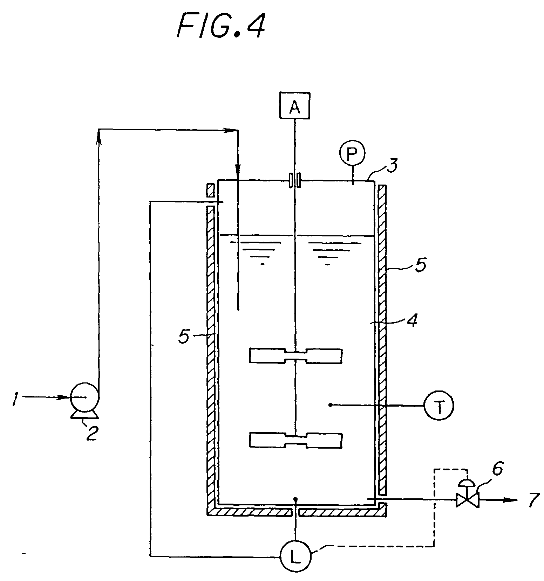

Example 1 was repeated to try to prepare continuously cyanonorbornene except that the pipe for supplying the raw material was introduced into the liquid in the vessel type reactor as shown in Fig. 4 for the reaction to prepare continuously cyanonorbornene. It became difficult to supply the raw material in 3 days after the initiation of the reaction, so that the operation was stopped. The inside of the reactor was inspected to find that a large amount of insoluble polymers were adhered to the inner wall of the supply pipe, especially at or around the surface of the outlet pipe for the raw material. The pipe was just before being clogged.

### Comparative Example 6

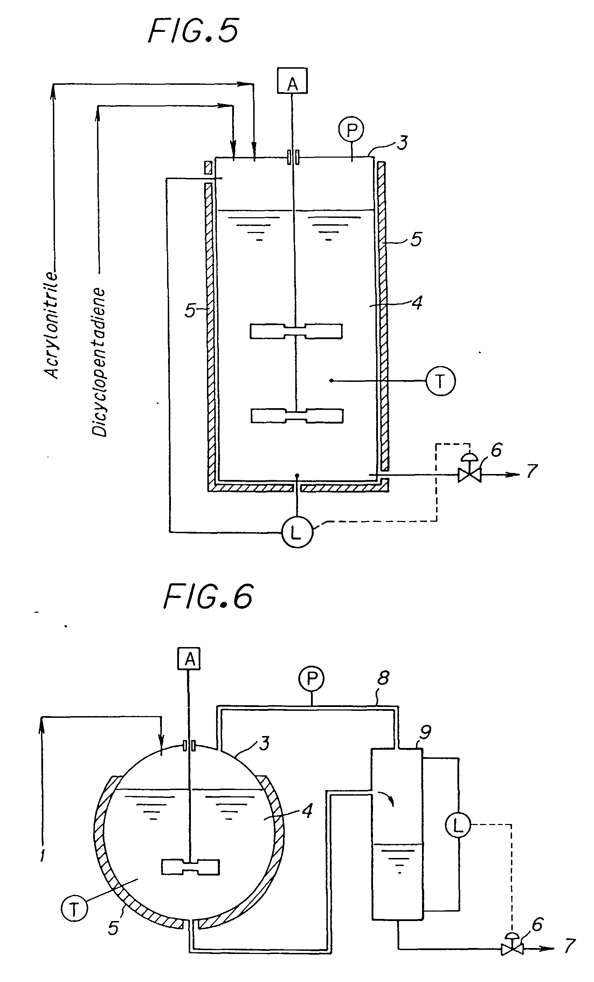

Example 1 was repeated to try to prepare continuously cyanonorbornene except that acrylonitrile and dicyclopentadiene, which were the raw materials, were independently supplied to the gaseous phase in the reactor as shown in Fig. 5 without being mixed with each other in advance. It became difficult to supply acrylonitrile in 2 days after the initiation of the reaction, so that the operation was stopped. The inside of the reactor was inspected to find that a large amount of insoluble polymers were adhered to the inner wall of the pipe for feeding acrylonitrile, especially at or around the surface of the outlet pipe for acrylonitrile.

### Comparative Example 7

Example 1 was repeated except that hydroquinone was used in place of N-nitrosodiphenylamine in the same amount. As a result, cyanonorbornene was continuously obtained with the average yield of 83% by mole. However, it became difficult to supply the raw material in 4 days after the initiation of the reaction, so that the operation was stopped. The inside of the reactor was inspected to find that a large amount of insoluble polymers were adhered to the inner wall of the pipe for feeding the raw material, especially at or around the surface of the outlet pipe for the raw material. The pipe was just before being clogged.

### Example 13

Example 1 was repeated except that the reactor used was a spherical type reactor of which essentials are shown in Fig. 6 and that the volume of the gaseous phase was 30% relative to the entire volume of the reactor. The reaction solution was flowed out to a gas-liquid separator to keep the liquid level in the reactor. Also in this example, the pressure in the reactor could be kept at 8 kg/cm²·G. The supplying amount of the raw material was not changed or fluctuated. Cyanonorbornene was obtained continuously with the average yield of 93% by mole. The operation was stopped in 50 days after the initiation of the reaction to inspect in detail the inside of the reactor, the inlet pipe for the raw material and the outlet pipe for the reaction solution. As a result, no insoluble polymer was found to be adhered.

### Example 14

A reactor used was a pressure autoclave made of stainless steel and equipped with a stirrer having the inner volume of 1,500 ml, the essentials of which are shown in Fig. 1. Before initiation of reaction, 1,015 ml of cyanocyclohexene was filled in the autoclave. The content of the reactor was heated to 150°C while stirring at the rotating speed of 600 rpm. Next, 1,3-butadiene and acrylonitrile were mixed in a pressurized vessel in a molar ratio of 1:1.2. N-nitrosodiphenylamine was added to the mixed solution such that the content thereof is 0.1% by weight to provide a raw material solution. The raw material solution was continuously supplied to the autoclave by means of a metering pump such that the residence time becomes 1 hour. During the reaction, the liquid level in the reactor was kept such that the volume of the gaseous phase over the reaction solution in the reactor is 30% relative to the entire volume of the reactor. In addition, nitrogen gas was fed to the reactor to adjust the pressure so as to be 65 kg/cm²·G in the autoclave. Furthermore, the temperature of the liquid was kept at 185°C for the continuous reaction.

As a result, cyanocyclohexene was continuously obtained with the average yield of 83% by mole based on 1,3-butadiene used as the raw material. The pressure in the reactor was not changed or fluctuated suddenly in this example. Furthermore, the supplying amount of the raw material was not changed. The operation was stopped in 15 days after the initiation of the reaction to inspect in detail the inside of the reactor, the inlet pipe for the raw material and the outlet pipe for the reaction solution. As a result, no insoluble polymer was found to be adhered.

### Example 15

A reactor used was a pressure autoclave made of stainless steel and equipped with a stirrer having the inner volume of 1,500 ml, the essentials of which are shown in Fig. 1. Before initiation of reaction, 1,015 ml of methylcyanocyclohexene was filled in the autoclave. The content of the reactor was heated to 150°C while stirring at the rotating speed of 600 rpm. Next, isoprene and acrylonitrile were mixed in a vessel cooled to 10°C in a molar ratio of 1:1.3. N-nitrosodiphenylamine was added to the mixed solution such that the content thereof is 0.1% by weight to provide a raw material solution. The raw material solution was continuously supplied to the autoclave by means of a metering pump such that the residence time becomes 2 hours. During the reaction, the liquid level in the reactor was kept such that the volume of the gaseous phase over the reaction solution in the reactor is 30% relative to the entire volume of the reactor. In addition, nitrogen gas was fed to the reactor to adjust the pressure so as to be 45 kg/cm²·G in the autoclave. Furthermore, the temperature of the liquid was kept at 185°C for the continuous reaction.

As a result, methylcyanocyclohexene was continuously obtained with the average yield of 85% by mole based on isoprene used as the raw material. The pressure in the reactor was not changed or fluctuated suddenly in this example. Furthermore, the supplying amount of the raw material was not changed. The operation was stopped in 15 days after the initiation of the reaction to inspect in detail the inside of the reactor, the inlet pipe for the raw material and the outlet pipe for the reaction solution. As a result, no insoluble polymer was found to be adhered.

### Comparative Example 8

Example 14 was repeated except that the pipe for supplying the raw material was introduced into the liquid in the vessel type reactor as shown in Fig. 4 to continuously prepare cyanocyclohexene. In this comparative example, it became difficult to supply the raw material in 1 day after the initiation of the reaction, so that the operation was stopped at that time and the inside of the pipe or the reactor was inspected. As a result, it was found that a large amount of insoluble polymer were adhered to the inner surface of the supply pipe, especially at or around the surface of the outlet pipe for the raw material. The pipe was just before being clogged.

### Comparative Example 9

Example 15 was repeated except that the reactor of which essentials are shown in Fig. 3 was used and that the reactor was all full of the reaction solution to prepare methylcyanocyclohexene. However, this example was suffered from considerable fluctuation of the pressure in the reactor with increase of the inner temperature. The pressure was suddenly fluctuated in the range of 35-55 kg/cm²·G. There was a great possibility of damaging the reactor and auxiliary facilities. It was thus found to be difficult to prepare methylcyanocyclohexene continuously in a safe manner.

## Claims

1. A process for preparing a Diels-Alder addition product of a conjugated diolefin and acrylonitrile comprising the steps of:
providing a vessel type reactor; increasing the pressure in a gaseous phase present therein using a gas which is inert to the reaction;
releasing a premixed solution of the conjugated diolefin, or a compound which produces the conjugated diolefin and acrylonitrile in the reactor through an upper portion of the reactor into the gaseous phase and dropping the mixed solution into a liquid phase to react;
reacting the conjugated diolefin and acrylonitrile at a temperature of 180°C to 200°C; and
removing reaction product from a lower portion of the reactor.

2. A process as claimed in claim 1, wherein the volume of the gaseous phase is within the range of 10-50% relative to the entire volume of the reactor.

3. A process as claimed in claim 1 or claim 2, wherein the pressure during the reaction is within the range of 6-70 kg/cm².G.

4. A process as claimed in any one of the preceding claims, wherein the gas which is inert to the reaction is nitrogen, argon, helium, methane, ethane, propane, n-butane or isobutane.

5. A process according to any one of the preceding claims, wherein the compound from which the conjugated diolefin is produced is dicyclopentadiene, and the pressure during the reaction is within the range of 6-30 kg/cm².G.

6. A process as claimed in claim 5, wherein the pressure during the reaction is within the range of 6-10 kg/cm².G.

7. A process according to any one of claims 1 to 4 wherein the conjugated diolefin is butadiene and the pressure during the reaction is within the range of 50-70 kg/cm².G.

8. A process as claimed in claim 7, wherein the gas which is inert to the reaction is nitrogen, argon, helium, methane or ethane.

9. A process according to any of claims 1 to 4, wherein the conjugated diolefin is isoprene and the pressure during the reaction is within the range of 30-50 kg/cm².G.

10. A process as claimed in claim 9, wherein the gas which is inert to the reaction is nitrogen, argon, helium, methane, ethane or propane.

11. A process according to any one of the previous claims wherein the reaction between the conjugated diolefin and acrylonitrile is carried out in the presence of an N-nitrosoamine compound or a p-phenylenediamine compound.

12. A process as claimed in claim 11, which is carried out in the presence of an N-nitrosoamine compound selected from N-nitrosodimethylamine, N-nitrosodiethylamine, N-nitrosomethylethylamine, N-nitrosodiphenylamine, N-nitrosobenzylaniline or 4-nitroso-N, N-dimethylaniline.

13. A process as claimed in claim 11, which is carried out in the presence of N-phenyl-N'-isopropyl-p-phenylenediamine.

## Patentansprüche

1. Verfahren zur Herstellung eines Diels-Alder-Additionsproduktes aus einem konjugierten Diolefin und Acrylnitril, folgende Schritte umfassend:
das Bereitstellen eines gefäßartigen Reaktors; das Erhöhen des Drucks in einer darin vorhandenen Gasphase unter Einsatz eines gegenüber der Reaktion inerten Gases;
das Einlassen einer vorgemischten Lösung des konjugierten Diolefins oder einer Verbindung, die das konjugierte Diolefin im Reaktor erzeugt, und von Acrylnitril durch einen oberen Abschnitt des Reaktors in die Gasphase und das Zutropfen der Mischlösung zu einer flüssige Phase zur Reaktion;
das Umsetzen des konjugierten Diolefins und Acrylnitrils bei einer Temperatur von 180 °C bis 200 °C; und
das Abziehen von Reaktionsprodukt aus einem unteren Abschnitt des Reaktors.

2. Verfahren nach Anspruch 1, worin das Volumen der Gasphase im Bereich von 10 bis 50 % des Gesamtvolumens des Reaktors liegt.

3. Verfahren nach Anspruch 1 oder 2, worin der Druck während der Reaktion im Bereich von 6 bis 70 kg/cm² liegt.

4. Verfahren nach einem der vorangegangenen Ansprüche, worin das gegenüber der Reaktion inerte Gas Stickstoff, Argon, Helium, Methan, Ethan, Propan, n-Butan oder Isobutan ist.

5. Verfahren nach einem der vorangegangenen Ansprüche, worin die Verbindung, aus der das konjugierte Diolefin erzeugt wird, Dicyclopentadien ist und der Druck während der Reaktion im Bereich von 6 bis 30 kg/cm² liegt.

6. Verfahren nach Anspruch 5, worin der Druck während der Reaktion im Bereich von 6 bis 10 kg/cm² liegt.

7. Verfahren nach einem der Ansprüche 1 bis 4, worin das konjugierte Diolefin Butadien ist und der Druck während der Reaktion im Bereich von 50 bis 70 kg/cm² liegt.

8. Verfahren nach Anspruch 7, worin das gegenüber der Reaktion inerte Gas Stickstoff, Argon, Helium, Methan oder Ethan ist.

9. Verfahren nach einem der Ansprüche 1 bis 4, worin das konjugierte Diolefin Isopren ist und der Druck während der Reaktion im Bereich von 30 bis 50 kg/cm² liegt.

10. Verfahren nach Anspruch 9, worin das gegenüber der Reaktion inerte Gas Stickstoff, Argon, Helium, Methan, Ethan oder Propan ist.

11. Verfahren nach einem der vorangegangenen Ansprüche, worin die Reaktion zwischen dem konjugierten Diolefin und Acrylnitril in Gegenwart einer N-Nitrosoamin-Verbindung oder einer p-Phenylendiamin-Verbindung durchgeführt wird.

12. Verfahren nach Anspruch 11, das in Gegenwart einer N-Nitrosoamin-Verbindung durchgeführt wird, die aus N-Nitrosodimethylamin, N-Nitrosodiethylamin, N-Nitrosomethylethylamin, N-Nitrosodiphenylamin, N-Nitrosobenzylanilin oder 4-Nitroso-N,N-dimethylanilin ausgewählt ist.

13. Verfahren nach Anspruch 11, das in Gegenwart von N-Phenyl-N'-isopropyl-p-phenylendiamin durchgeführt wird.

## Revendications

1. Un procédé de préparation d'un produit d'addition Diels-Alder d'une dioléfine conjuguée et d'un acrylonitrile comprenant les étapes de :
prévoir un récipient formant réacteur ; augmenter la pression dans une phase gazeuse présente à l'intérieur en utilisant un gaz qui est inerte à la réaction ;
libérer une solution prémélangée de la dioléfine conjuguée, ou d'un composé qui produit la dioléfine conjuguée et l'acrylonitrile, dans le réacteur par l'intermédiaire d'une partie supérieure du réacteur dans la phase gazeuse et faire tomber la solution mélangée dans une phase liquide en vue d'une réaction ;
faire réagir la dioléfine conjuguée et l'acrylonitrile à une température de 180°C à 200°C ; et
retirer le produit de réaction depuis une partie inférieure du réacteur.

2. Un procédé tel que revendiqué à la revendication 1, dans lequel le volume de la phase gazeuse est à l'intérieur de la gamme de 10 à 50% par rapport au volume entier du réacteur.

3. Un procédé tel que revendiqué à la revendication 1 ou la revendication 2, dans lequel la pression pendant la réaction est à l'intérieur de la gamme de 6 à70 kg/cm².G.

4. Un procédé tel que revendiqué dans une quelconque des revendications précédentes, dans lequel le gaz qui est inerte à la réaction est l'azote, l'argon, l'hélium, le méthane, l'éthane, le propane, le n-butane ou l'isobutane.

5. Un procédé selon l'une quelconque des revendications précédentes, dans lequel le composé à partir duquel est produite la dioléfine conjuguée est le dicyclopentadiène, et la pression pendant la réaction est à l'intérieur de la gamme de 6 à 30 kg/cm².G.

6. Un procédé tel que revendiqué à la revendication 5, dans lequel la pression pendant la réaction est à l'intérieur de la gamme de 6 à 10 kg/cm².G.

7. Un procédé selon l'une quelconque des revendications 1 à 4, dans lequel la dioléfine conjuguée est le butadiene et la pression pendant la réaction est à l'intérieur de la gamme de 50 à 70 kg/cm².G.

8. Un procédé tel que revendiqué à la revendication 7, dans lequel le gaz qui est inerte à la réaction est l'azote, l'argon, l'hélium, le méthane ou l'éthane.

9. Un procédé selon l'une quelconque des revendications 1 à 4, dans lequel, la dioléfine conjuguée est l'isoprène et la pression pendant la réaction est à l'intérieur de la gamme de 30 à 50 kg/cm².G.

10. Un procédé tel que revendiqué à la revendication 9, dans lequel le gaz qui est inerte à la réaction est l'azote, l'argon, l'hélium, le méthane, l'éthane ou le propane.

11. Un procédé selon l'une quelconque des revendications précédentes dans lequel la réaction entre la dioléfine conjuguée et l'acrylonitrile est mis en oeuvre en présence d'un composé de N-nitrosoamine ou d'un composé de p-phénylènediamine.

12. Un procédé tel que revendiqué à la revendication 11, qui est mis en oeuvre en présence d'un composé de N-nitrosoamine choisi parmi la N-nitrosodiméthylamine, la N-nitrosodiéthylamine, la N-nitrosométhyléthylamine, la N-nitrosodiphénylamine, la N-nitrosobenzylaniline ou la 4-nitroso-N, N-diméthylaniline.

13. Un procédé tel que revendiqué à la revendication 11, qui est mis en oeuvre en présence de N-phényl-N'-isopropyl-p-phénylènediamine.
